# FASCICULE DE BREVET EUROPEEN

(11) **EP 0 742 194 B1**
(45) Date de publication et mention de la délivrance du brevet: **04.08.1999**
(21) Numéro de dépôt: 96400998.9
(22) Date de dépôt: 10.05.1996
(51) Int. Cl.: C07C 37/14, C07C 39/02, C07C 39/08, C07D 311/72

(54) **Procédé de préparations de phénols substitués**
Verfahren zur Herstellung von substituierten Phenolen
Process for the preparation of substituted phenols

(30) Priorité: 10.05.1995 FR 9505509
(43) Date de publication de la demande: 13.11.1996
(73) Titulaire: RHONE-POULENC NUTRITION ANIMALE, 92160 Antony (FR)
(72) Inventeur: Ancel, Jean-Erick, 69230 Saint Genis Laval (FR); Bienayme, Hugues, 69002 Lyon (FR); Meilland, Pierre, 69360 Chaponost (FR)
(74) Mandataire: Le Pennec, Magali

(56) Documents cités:
- WO-A-90/01554
- US-A- 4 594 460
- CHEMICAL ABSTRACTS, vol. 93, no. 17, 27 Octobre 1980 Columbus, Ohio, US; abstract no. 168127r, page 664; XP002010962 & JP-A-55 015 411 (MITSUI PETROCHEMICAL INDUSTRIES) 2 Février 1980
- CHEMICAL ABSTRACTS, vol. 90, no. 23, 4 Juin 1979 Columbus, Ohio, US; abstract no. 187181h, page 685; XP002010963 & JP-A-53 149 979 (TAKASAGO PERFUMERY COMPANY) 27 Décembre 1978

## Description

La présente invention concerne un nouveau procédé de préparation de phénols substitués. Elle concerne plus particulièrement la condensation de phénols porteurs d'un ou plusieurs substituants alkyle avec un dérivé du butadiène comportant au moins six atomes de carbone. Elle concerne aussi la cyclisation sous forme de chromanes des produits obtenus lors de cette condensation.

Il est connu selon le brevet US 4 594 460 de condenser un phénol substitué par un ou plusieurs radicaux choisis parmi les halogènes, les groupes hydroxyles événtuellement sous forme d'éther ou d'ester, les groupes alkyl, nitro, aldéhyde éventuellement sous forme d'acétal, acétyl, benzyl, amino, alkylamino, dialkylamino ou alkyloxycarbonyl avec un dérivé du butadiène contenant 5 à 24 atomes de carbone. Cette condensation est réalisée, selon ce brevet, en présence d'un catalyseur à base de rhodium et d'une phosphine soluble dans l'eau.

Il est apparu que lorsque l'on voulait appliquer cette réaction à la condensation d'un phénol porteur d'un radical hydroxy et de plusieurs radicaux méthyle, par exemple au triméthylphénol ou à la triméthylhydroquinone avec un dérivé du butadiène comportant au moins six atomes de carbone, cette réaction ne donnait pas le produit attendu. La reproduction de cette réaction est incluse dans cette demande.

Il est encore connu selon le brevet japonais publié sous le numéro JP 55-15411 de faire réagir un diméthoxyphénol avec l'isoprène en présence d'un catalyseur à base de rhodium et d'une triphénylphosphine. Lorsque l'on applique ces conditions de réaction à la condensation du dérivé du butadiène comportant plus de six atomes de carbone tel que le myrcène avec la triméthylhydroquinone aucun produit de réaction n'est formé.

Même après la lecture de ces deux antériorités relatives à la substitution d'un phénol par un dérivé du butadiène, le problème restait entier en ce qui concerne la condensation d'un phénol non activé par plusieurs groupes alkoxy et un dérivé du butadiène comportant au moins six atomes de carbone.

La présente invention a permis d'atteindre ce premier objectif c'est à dire la condensation d'un phénol ou d'un diphénol substitué par au moins un groupe alkyle avec un dérivé du butadiène comportant au moins six atomes de carbone.

Elle concerne un procédé de préparation de phénols substitués de formule générale (I) 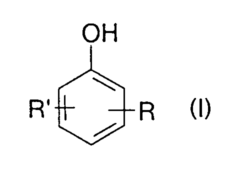 dans laquelle R représente un ou plusieurs groupes, identiques ou différents, choisis parmi les radicaux hydrogène, hydroxy ou alkyle ayant 1 à 6 atomes de carbone et R' représente un radical choisi parmi les radicaux de formules (II) suivantes 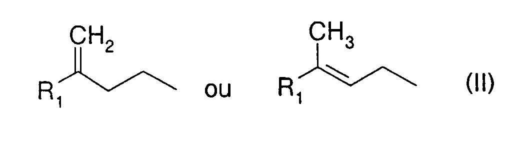 dans lesquelles le radical R1 représente un radical alkyl ou alkylène éventuellement substitué, le radical de formule (II) contenant au moins 6 atomes de carbone caractérisé en ce qu'on condense dans un milieu monophasique le phénol de formule (III) 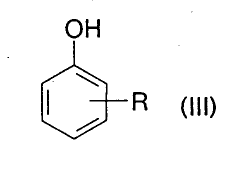 dans laquelle R a la même signification que dans la formule (I) avec un dérivé du butadiène de formule (IV) suivante: 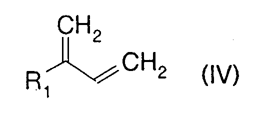 dans laquelle R1 a la même signification que dans les formules (II) en présence d'un catalyseur à base de rhodium à l'état d'oxydation (+1) et d'une diphosphine soluble dans un solvant organique aprotique et en présence d'une base.

Parmi les dérivés du butadiène de formule (IV) on préfère utiliser les composés polyéniques et de préférence le myrcène, le springène et le farnésène. Parmi les dérivés de formule (III) on préfère utiliser les composés pour lesquels R représente plusieurs radicaux alkyle et tout particulièrement le triméthylphénol et la triméthyl hydroquinone.

Le catalyseur de réaction à base de rhodium est de préférence un sel organique du rhodium ce dernier étant à l'état d'oxydation +1, choisi notamment parmi [Rh Cl (P(C₆H₅)₃)₂]₂, Rh Cl (CO) (P(C₆H₅)₃)₂ et [Rh Cl COD)]₂. Il est évident que l'homme de l'art pourra mettre en oeuvre un dérivé du rhodium à l'état d'oxydation supérieur mais il devra ajouter un élement réducteur avant la mise en oeuvre du catalyseur dans la réaction envisagée.

La diphosphine soluble dans un solvant organique aprotique est de préférence choisie parmi les diphosphines bidentates présentant une chaine hydrocarbonée entre les deux atomes de phosphore comprenant 3 à 5 atomes de carbone. On peut citer parmi les phosphines préférées la 1,4 bis(diphénylphosphino) butane, la 1,3 bis(diphénylphosphino) propane, la 1,2 bis(diphénylphosphino méthylène) cyclobutane, le 1,5 bis diphénylphosphino pentane). On préfère utiliser le 1,4 bis(diphénylphosphino) butane de formule : 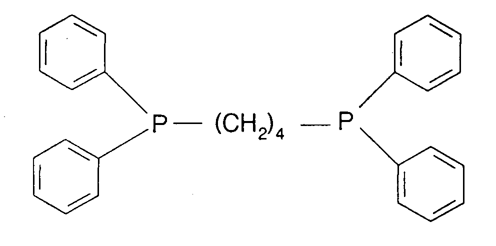 ou le 1,2 bis(diphosphino méthylène) cyclobutane de formule :

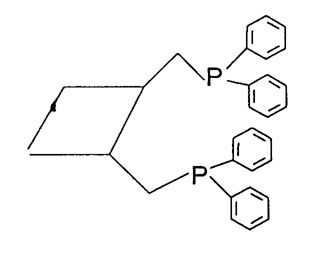

La présente invention concerne plus particulièrement la condensation du β springène ou du myrcène avec la triméthylhydroquinone en présence d'un catalyseur à base de rhodium à l'état d'oxydation (+1) et de bis diphénylphosphino butane ou cyclobutane.

Selon une meilleure manière de mise en oeuvre de l'invention, le rapport molaire du catalyseur à base de rhodium par rapport au dérivé du butadiène est de préférence compris entre (0,1 % et 10 %). Le rapport molaire du dérivé du butadiène au phénol est de préférence compris entre 0,2 et 2.

Tout type de solvant peut être utilisé tel que l'eau ou les solvants organiques ou un mélange des deux. Selon un meilleur moyen de mettre en oeuvre l'invention, le solvant utilisé est de préférence aprotique, il solubilisera alors notamment le phénol et le dérivé du butadiène, ainsi les solvants aromatiques, les éthers, les cétones et les esters sont utilisables dans le cadre de la présente invention. On préfère parmi ces solvants utiliser le toluène, le diméthoxyéthane et l'acétate d'isopropyle (ACIP).

Pour catalyser la réaction, il est avantageux d'ajouter une base de préférence insoluble dans le milieu de réaction. On peut ainsi utiliser les carbonates alcalins tels que les carbonates de sodium ou de potassium. Cette base peut être ajouté sous forme solide ou sous forme d'une solution aqueuse.

La température de réaction est de préférence comprise entre 30°C et 150°C et la durée de réaction varie entre quelques heures et une journée.

Selon une deuxième partie de l'invention le dérivé obtenu par condensation entre le phénol et le dérivé du butadiène est cyclisé en présence d'un catalyseur choisi parmi l'acide paratoluène sulfonique, le triflate de scandium, le dichlorométhylaluminium, le triflate d'ytterbium, le triflate de lanthane, le triflate stanneux, le chlorure de zinc et le dichloroéthoxyaluminium. On obtient un dérivé de formule générale (VI) suivante: 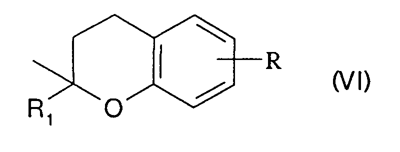 dans laquelle R₁ représente un radical alkyle ou alkényle contenant au moins deux atomes de carbone, ayant la même signification que dans la formule (II) et R représente le ou les mêmes radicaux que dans la formule (I).

Le dérivé obtenu peut ensuite être hydrogéné.

Selon une manière préférée de mettre en oeuvre l'invention, on condense la triméthylhydroquinone avec le β springène pour former le dérivé suivant de formule (V): 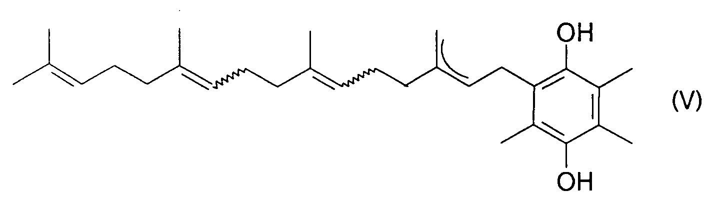 que l'on cyclise avec de préférence un catalyseur choisi parmi le dichlorométhylaluminium et l'acide paratoluène sulfonique pour donner le composé de formule générale (VI) ayant la structure suivante (VII) : 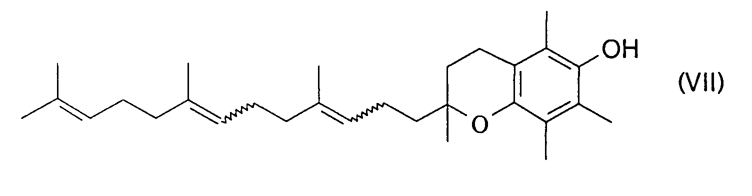 que l'on hydrogène ensuite pour obtenir la vitamine E. L'hydrogénation est réalisée de préférence en présence d'un catalyseur à base de palladium. L'acétate de tocophérol, produit commercial est ensuite obtenu par acétylation du dérivé obtenu à l'étape précédente.

L'invention sera plus complétement décrite à l'aide des exemples suivants qui ne doivent pas être considérés comme limitatifs de l'invention.

### EXEMPLES

Au cours des exemples suivants on entend par taux de transformation (TT) le rapport entre le composé transformé et le composé introduit. On entend par rendement le rapport molaire entre le produit obtenu et le produit introduit. Le rendement est égal au produit entre le taux de transformation et le rendement sur le produit transformé.

### Mode opératoire type

Dans un réacteur de 50 ml muni d'une agitation magnétique, d'un thermomètre et d'un réfrigérant, on charge :
- 15 mmoles de diène
- 30 mmoles de composé phénolique
- 3 mmoles de carbonate de potassium
- 0,45 mmoles de diphénylphosphinobutane
- 0,15 mmoles de (RhClCOD)₂
- puis 15 ml de toluène dégazé.

On purge à l'argon puis on porte au reflux à 120°C pendant 8 heures. On revient ensuite à 20°C, et on reprend le milieu réactionnel par 15 ml d'eau et 15 ml d'éther éthylique. La phase aqueuse est lavée par 15 ml d'éther éthylique et les phases éthérées sont rassemblées, lavées par 15 ml d'eau, séchées sur MgSO₄ filtrées et concentrées.

La taux de transformation du diène est quantitatif (analyse par chromatographie gaz, par chromatographie liquide, par chromatographie couche mince et par RMN¹H). L'excès de composé phénolique est enlevé par lavage à la soude 1N. Il peut être recyclé par extraction acide.

### COUPLES DIENE/COMPOSE PHENOLIQUE

| Exemples | DIENE | COMPOSE PHENOLIQUE | RENDEMENT ISOLE | TEMPS heures |
|---|---|---|---|---|
| 1 | Myrcène | triméthyl hydroquinone | 85 % | 8 |
| 2 | Myrcène | 2,3,5 triméthyl phénol | 90 % | 8 |
| 3 | Myrcène | 2,6 diméthyl phénol | 74 % | 8 |
| 4 | β springène | triméthyl hydroquinone | 85 % | 8 |
| 5 | Myrcène | Phénol | 72 % | 8 |
| 6 | β springène | 2,3,5 triméthyl phénol | 67 % | 8 |

### NATURE DE LA PHOSPHINE

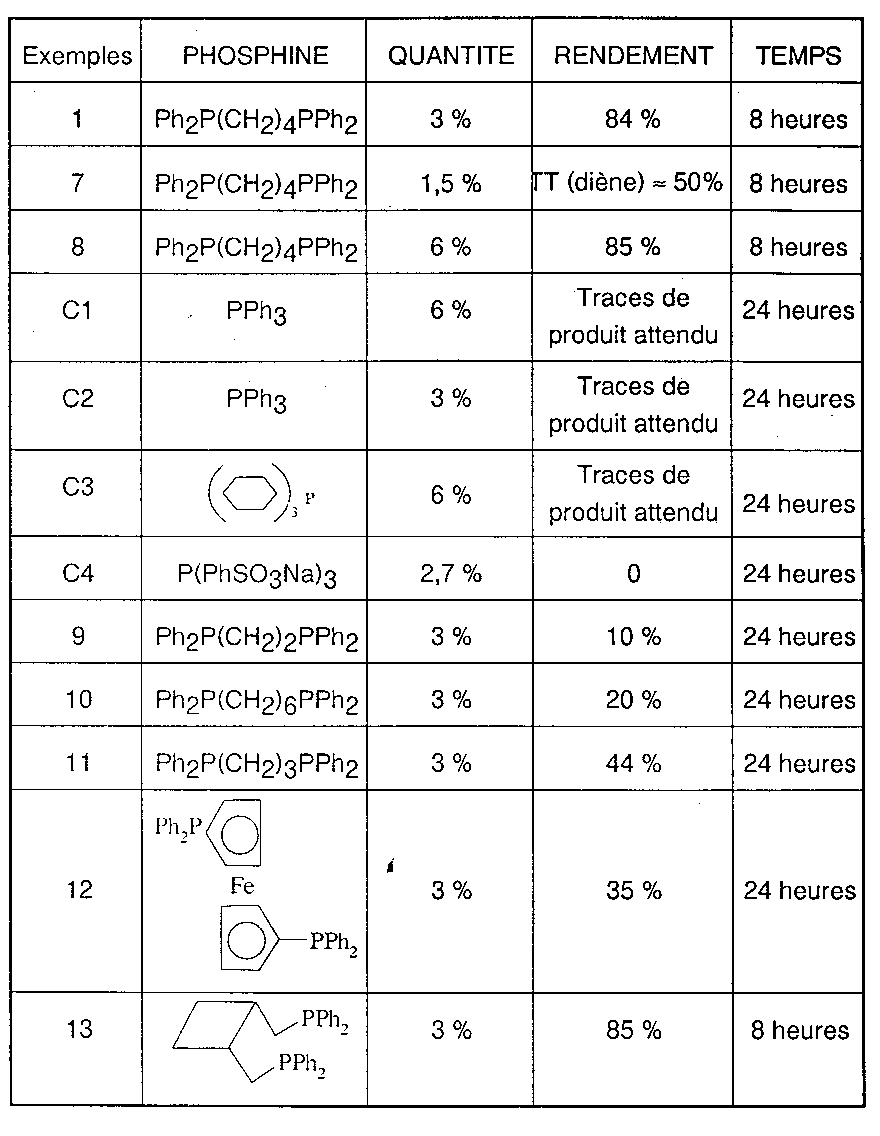

### SOURCE DE RHODIUM

On reproduit l'exemple 1 avec les catalyseurs suivants :
- [Rh Cl (P(C₆H₅)₃)₂]₂
- Rh Cl (CO) (P(C₆H₅)₃)₂
- [Rh Cl (COD)]₂

Les résultats de la condensation sont exprimés dans le tableau suivant : Myrcène + TMHQ :

| | | |
|---|---|---|
| (Rh Cl COD)₂ | 85 % | 8 à 16 heures |
| Rh Cl (CO) (PPh₃)₂ | 85 % | 12 heures |
| (Rh Cl (PPh₃)₂)₂ | 85 % | 12 heures |

### EXEMPLES 14 A 24 VARIATION DE LA NATURE DU SOLVANT

| EXEMPLES | REACTIFS | SOLVANT | BASE | RESULTAT |
|---|---|---|---|---|
| 14 | Myrcène | H₂O 24 h à 120°C | tampon pH 10 ou K₂CO₃ 1 eq | RR = 70 % |
| | | | | TT(C₉) = 72 % |
| 15 | Myrcène | NMP 24 h à 120°C | 1 eq K₂CO₃ | TT = 28 % |
| 16 | Myrcène | Toluène/H₂O 8/15 | Tampon pH 10 | TT(C₉) = 83 |
| | | | | % RR = 71 % |
| 17 | Myrcène | NMP/H₂O 8/15 | Tampon pH 10 | TT(C₉) = 58 |
| | | | | % RR = 31 % |
| 18 | Myrcène (2 eq TMHQ) | DME 17 h à 85°C | 1 eq K₂CO₃ | TT(C₁₀) = |
| | | | | 100% RR dosé ≈ 95% |
| 19 | β springène + 2 eq TMHQ | DME 8 h à reflux | 0,2 eq | RR dosé <20 % |
| 20 | β springène + 2 eq TMHQ | MIBK 8 h à reflux | 0,2 eq | RR dosé <20 % |
| 21 | β springène + 2 eq TMHQ | ACIP 16 h à reflux | 0,2 eq | RR dosé = 80 % |

### EXEMPLES 22 A 25

### ESSAI DE CYCLISATION

On met en oeuvre 25,2 g de dérivé phénolique en C₂₉ obtenu lors de la condensation du β springène avec la triméthylhydroquinone; 1,58 ml d'une solution molaire dans l'hexane de CH₃AlCl₂. On charge dans un ballon tricol sous agitation magnétique le dérivé phénolique dans 20 ml de toluène, on ajoute la solution hexanique de CH₃AlCl₂. On laisse réagir 24 heures à température ambiante puis on chauffe au reflux. On obtient le dérivé cyclisé de formule (VII) avec un rendement de 78 %. On purifie le produit par chromatographie sur silice puis on l'hydrogène en présence de 2% molaire de Pd/C puis on acétyle le dérivé hydrogèné en présence d'anhydride acétique, de triéthylamine, de diméthylaminopyridine dans l'hexane.

On obtient l'acétate de tocophérol avec un rendement en produit isolé sur le béta springène introduit de 47 %. On reproduit l'exemple précédent avec des catalyseurs de cyclisation de nature différente.

| EXEMPLES | Catalyseur de cyclisation Rapport molaire/ C₂₉ | Rendement en produit de formule (VI)/(V) | Rendement en acétate tocophérol /β srpingène |
|---|---|---|---|
| 22 | CH₃AlCl₂ [0,12] | 78 % | 47 % |
| 23 | Sc(CF₃SO₃)₃ [0,12] | 67 % | 22 % |
| 24 | EtO AlCl₂ [0,3] | 75 % | 29 % |
| 25 | acide paratoluène sulfonique[0,1] | 83 % | 34 % |

## Revendications

1. Procédé de préparation de phénols substitués de formule générale (I) 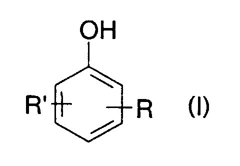 dans laquelle R représente un ou plusieurs groupes, identiques ou différents, choisis parmi les radicaux hydrogène, hydroxy ou alkyle ayant 1 à 6 atomes de carbone et R' représente un radical choisi parmi les radicaux de formules (II) suivantes 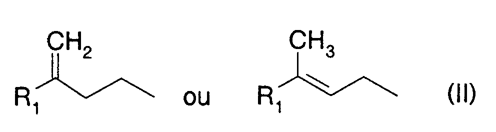 dans lesquelles le radical R1 représente un radical alkyl ou alkylène éventuellement substitué, le radical de formule (II) contenant au moins 6 atomes de carbone caractérisé en ce qu'on condense dans un milieu monophasique le phénol de formule (III) 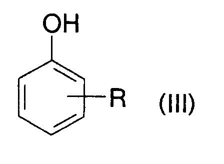 dans laquelle R a la même signification que dans la formule (I) avec un dérivé du butadiène de formule (IV) suivante: 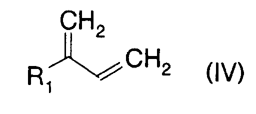 dans laquelle R1 a la même signification que dans les formules (II) en présence d'un catalyseur à base de rhodium à l'état d'oxydation +1 et d'une diphosphine soluble dans un solvant organique aprotique et en présence d'une base.

2. Procédé selon la revendication 1 caractérisé en ce que le dérivé du butadiène de formule (IV) est choisi parmi les composés polyéniques et de préférence parmi le myrcène, le springène et le farnésène.

3. Procédé selon la revendication 1 caractérisé en ce que le rhodium mis en oeuvre est un sel organique du rhodium choisi parmi [Rh Cl (P(C₆H₅)₃)₂]₂, Rh Cl (CO) (P(C₆H₅)₃)₂ et [Rh Cl COD)]₂.

4. Procédé selon la revendication 1 caractérisé en ce que le phénol de formule (III) est choisi de préférence parmi le triméthylphénol et la tétraméthyl hydroquinone.

5. Procédé selon la revendication 1 caractérisé en ce que la diphosphine soluble dans un solvant organique aprotique est de préférence choisie parmi les diphosphines bidentates présentant une chaine hydrocarbonée entre les deux atomes de phosphore comprenant 3 à 5 atomes de carbone.

6. Procédé selon la revendication 5 caractérisé en ce que la phosphine utilisée est le diphénylphosphino butane de formule :

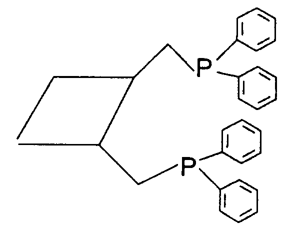

7. Procédé selon la revendication 1 caractérisé en ce qu'on prépare le dérivé de formule (V) suivante : 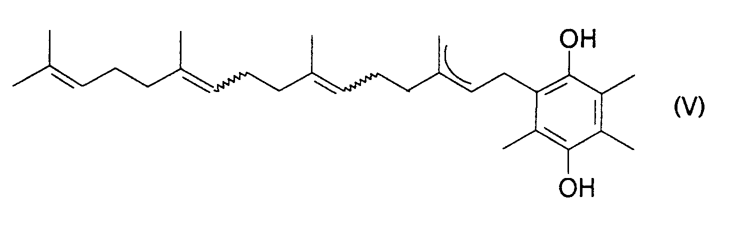 par condensation du springène et de la triméthylhydroquinone en présence de la phosphine selon la revendication 4 et de (Rh Cl COD)₂ dans le toluène.

8. Procédé de préparation selon la revendication 1 caractérisé en ce qu'on met en contact le phénol de formule (III) et le dérivé du butadiène de formule (IV) selon un rapport molaire du dérivé du butadiène au phénol sur compris entre 0,2 et 2.

9. Procédé de préparation de la vitamine E caractérisé en ce que l'on prépare le dérivé de formule (V) selon l'une quelconque des revendications 1 à 8 et en ce que l'on cyclise ledit dérivé de formule (V) 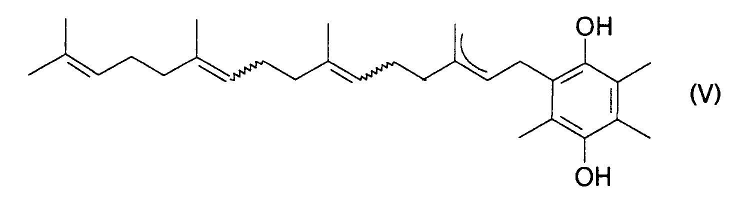 en présence d'un catalyseur choisi parmi l'acide paratoluène sulfonique, le triflate de scandium, le dichlorométhylaluminium, le triflate d'ytterbium, le triflate de lanthane, le triflate stanneux, le chlorure de zinc et le dichloroéthoxyaluminium puis on hydrogène le dérivé obtenu lors de la cyclisation.

## Claims

1. Process for the preparation of substituted phenols of general formula (I) 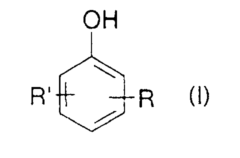 in which R represents one or more groups, which are identical or different, chosen from hydrogen, hydroxyl radicals or alkyl radicals having 1 to 6 carbon atoms and R' represents a radical chosen from the radicals of the following formulae (II) 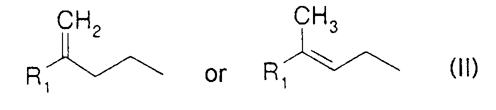 in which the radical R₁ represents an optionally substituted alkylene or alkyl radical, the radical of formula (II) containing at least 6 carbon atoms, characterized in that the phenol of formula (III) 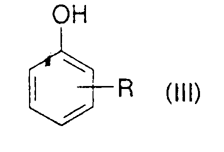 in which R has the same meaning as in the formula (I) is condensed in a single-phase medium with a butadiene derivative of the following formula (IV): 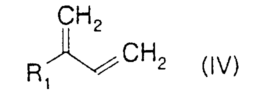 in which R₁ has the same meaning as in the formulae (II), in the presence of a catalyst based on rhodium with the oxidation number +1 and of a diphosphine soluble in an aprotic organic solvent and in the presence of a base.

2. Process according to claim 1, characterized in that the butadiene derivative of formula (IV) is chosen from polyene compounds and preferably from myrcene, springene and farnesene.

3. Process according to claim 1, characterized in that the rhodium used is an organic salt of rhodium chosen from [Rh Cl (P(C₆H₅)₃)₂]₂, Rh Cl (CO) (P(C₆H₅)₃)₂ and [Rh Cl (COD)]₂.

4. Process according to claim 1, characterized in that the phenol of formula (III) is chosen preferably from trimethylphenol and tetramethylhydroquinone.

5. Process according to claim 1, characterized in that the diphosphine soluble in an aprotic organic solvent is preferably chosen from the bidentate diphosphines having a hydrocarbon chain between the two phosphorus atoms comprising 3 to 5 carbon atoms.

6. Process according to claim 5, characterized in that the phosphine used is the diphenylphosphinobutane of formula:

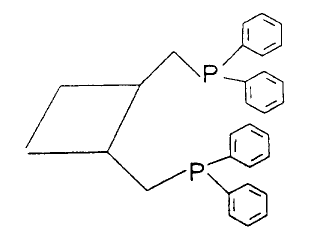

7. Process according to claim 1, characterized in that the derivative of the following formula (V): 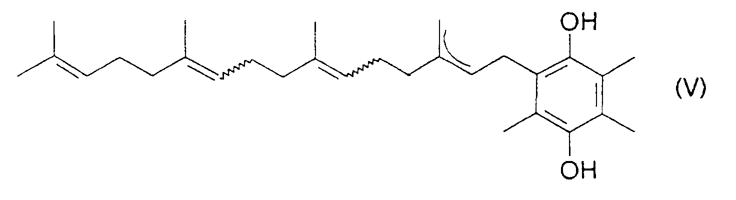 is prepared by condensation of springene with trimethylhydroquinone in the presence of phosphine according to claim 4 and of (RhClCOD)₂ in toluene.

8. Process of preparation according to claim 1, characterized in that the phenol of formula (III) and the butadiene derivative of formula (IV) are brought into contact in a molar ratio of the butadiene derivative to the phenol of between 0.2 and 2.

9. Process for the preparation of vitamin E, characterized in that the derivative of formula (V) is prepared according to any one of claims 1 to 8, and in that the said derivative of formula (V) 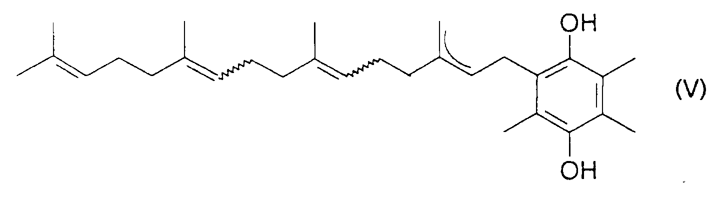 is cyclized in the presence of a catalyst chosen from paratoluenesulphonic acid, scandium triflate, dichloromethylaluminium, ytterbium triflate, lanthanum triflate, tin (II) triflate, zinc chloride, dichloroethoxyaluminium and then the derivative obtained during the cyclization is hydrogenated.

## Patentansprüche

1. Verfahren zur Herstellung von substituierten Phenolen der allgemeinen Formel (I) 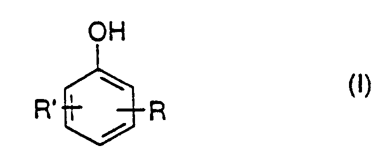 in der R eine oder mehrere gleiche oder verschiedene Gruppen darstellt, die ausgewählt sind unter den Wasserstoff-, Hydroxy- oder Alkylresten mit 1 bis 6 Kohlenstoffatomen, und R' einen Rest darstellt, der ausgewählt ist unter den Resten der folgenden Formeln (II) 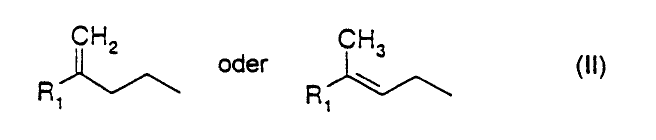 in denen der Rest R1 einen gegebenenfalls substituierten Alkyl- oder Alkylenrest darstellt, wobei der Rest der Formel (II) wenigstens 6 Kohlenstoffatome enthält, dadurch gekennzeichnet, daß man in einem monophasigen Medium das Phenol der Formel (III) 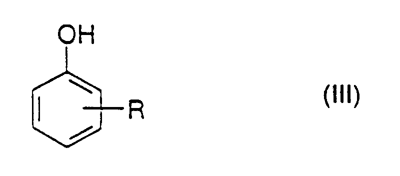 in der R die gleiche Bedeutung wie in der Formel (I) hat, mit einem Butadienderivat der folgenden Formel (IV): 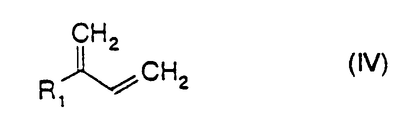 in der R1 die gleiche Bedeutung wie in den Formeln (II) hat, in Gegenwart eines Katalysators auf der Basis von Rhodium im Oxidationszustand +1 und eines Diphosphins, das in einem aprotischen organischen Lösungsmittel löslich ist, und in Gegenwart einer Base kondensiert.

2. Verfahren gemäß Anspruch 1, dadurch gekennzeichnet, daß das Butadienderivat der Formel (IV) unter den Polyenverbindungen und vorzugsweise unter Myrcen, Springen und Famesen ausgewählt ist.

3. Verfahren gemäß Anspruch 1, dadurch gekennzeichnet, daß das eingesetzte Rhodium ein organisches Rhodiumsalz ist, das unter [Rh Cl (P(C₆H₅)₃)₂]₂, Rh Cl (CO) (P(C₆H₅)₃)₂ und [Rh Cl COD]₂ ausgewählt ist.

4. Verfahren gemäß Anspruch 1, dadurch gekennzeichnet, daß das Phenol der Formel (III) vorzugsweise unter Trimethylphenol und Tetramethylhydrochinon ausgewählt ist.

5. Verfahren gemäß Anspruch 1, dadurch gekennzeichnet, daß das in einem aprotischen organischen Lösungsmittel lösliche Diphosphin vorzugsweise unter den zweizähnigen Diphosphinen, die zwischen den beiden Phosphoratomen eine Kohlenwasserstoffkette aufweisen, die 3 bis 5 Kohlenstoffatome umfaßt, ausgewählt ist.

6. Verfahren gemäß Anspruch 5, dadurch gekennzeichnet, daß das verwendete Phosphin das Diphenylphosphinobutan der Formel: 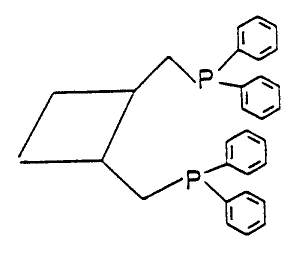 ist.

7. Verfahren gemäß Anspruch 1, dadurch gekennzeichnet, daß man das Derivat der folgenden Formel (V): 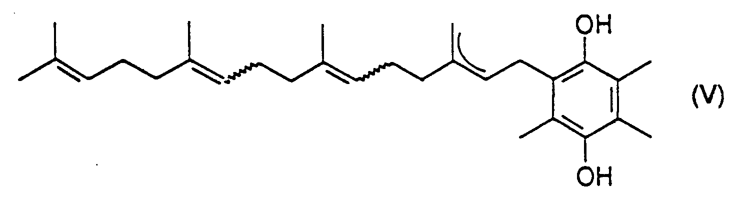 durch Kondensation von Springen und Trimethylhydrochinon in Gegenwart des Phosphins gemäß Anspruch 4 und (Rh Cl COD)₂ in Toluol herstellt.

8. Herstellungsverfahren gemäß Anspruch 1, dadurch gekennzeichnet, daß man das Phenol der Formel (III) und das Butadienderivat der Formel (IV) gemäß einem Molverhältnis Butadienderivat zu Phenol zwischen 0,2 und 2 in Kontakt bringt.

9. Verfahren zur Herstellung von Vitamin E, dadurch gekennzeichnet, daß man das Derivat der Formel (V) gemäß einem der Ansprüche 1 bis 8 herstellt, und dadurch, daß man besagtes Derivat der Formel (V) 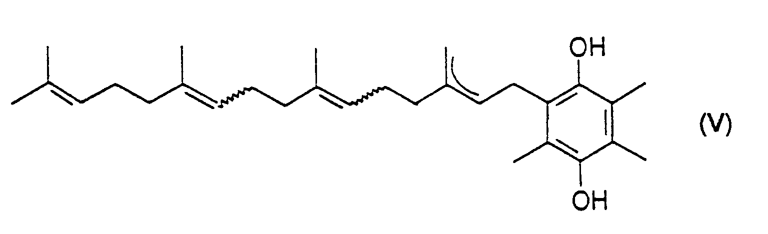 in Gegenwart eines Katalysators, der unter para-Toluolsulfonsäure, Scandiumtriflat, Dichlormethylaluminium, Ytterbiumtriflat, Lanthantriflat, Zinntriflat, Zinkchlorid und Dichlorethoxyaluminium ausgewählt ist, cyclisiert, man dann das bei der Cyclisierung erhaltene Derivat hydriert.
